(19) Europäisches Patentamt / European Patent Office / Office européen des brevets

(11) **EP 3 513 178 B1**

(12) **EUROPÄISCHE PATENTSCHRIFT**

(45) Veröffentlichungstag und Bekanntmachung des Hinweises auf die Patenterteilung:
**27.04.2022 Patentblatt 2022/17**

(21) Anmeldenummer: **17751721.6**

(22) Anmeldetag: **09.08.2017**

(51) Internationale Patentklassifikation (IPC):
*G01N 25/18* *(2006.01)*    *G01N 33/22* *(2006.01)*
*G01F 1/684* *(2006.01)*    *G01F 1/698* *(2006.01)*

(52) Gemeinsame Patentklassifikation (CPC):
**G01N 33/225; G01F 1/6845; G01F 1/6986; G01N 25/18**

(86) Internationale Anmeldenummer:
**PCT/EP2017/070225**

(87) Internationale Veröffentlichungsnummer:
**WO 2018/050371 (22.03.2018 Gazette 2018/12)**

(54) **GASZÄHLER**

GAS METER

COMPTEUR À GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.09.2016 DE 102016117215**

(43) Veröffentlichungstag der Anmeldung:
**24.07.2019 Patentblatt 2019/30**

(73) Patentinhaber: **Bundesrepublik Deutschland, vertreten durch das Bundesmisterium für Wirtschaft und Energie, endvertreten durch den Präsidenten der PTB 38116 Braunschweig (DE)**

(72) Erfinder: **HAMMERSCHMIDT, Ulf 38112 Braunschweig (DE)**

(74) Vertreter: **Gramm, Lins & Partner Patent- und Rechtsanwälte PartGmbB Theodor-Heuss-Straße 1 38122 Braunschweig (DE)**

(56) Entgegenhaltungen:
WO-A1-01/18500    WO-A1-2004/065915
DE-A1-102014 008 284    DE-T2- 69 817 875
US-A1- 2009 049 907

EP 3 513 178 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Gaszähler gemäß Anspruch 1.

Ein Gaszähler gemäß des Oberbegriffs von Anspruch 1 ist aus der DE 10 2014 008 284 A1 bekannt. Dort ist beschrieben, dass mittels eines solchen Gaszählers auch die Zusammensetzung des Gasgemischs ermittelt werden kann.

Aus der DE 698 17 875 T2 ist eine Vorrichtung zum Messen der Fluidgeschwindigkeit eines Fluids bekannt, bei dem ein Heizelement und mindestens zwei beabstandete Sensorelemente vorgesehen sind, wobei die Sensorelemente in unterschiedlichen Entfernungen von dem Heizelement angeordnet sind. Das Heizelement und die Sensorelemente sind innerhalb des Fluids angeordnet. Ein zeitlich veränderliches Eingangssignal wird an das Heizelement ausgegeben, wodurch sich das umgebende Fluid erwärmt. Gemessen wird die Durchgangszeit, welche die Temperaturstörung benötigt, um von dem Heizelement zu dem ausgewählten Sensorelementen zu gelangen. Aus den Durchgangszeiten lässt sich die Fluidgeschwindigkeit weitgehend unabhängig von den Fluideigenschaften berechnen.

Die US 2009/0049907 A1 beschreibt ein mikroelektromechanisches System zum Messen der Fluidgeschwindigkeit eines Fluids, bei dem ebenfalls eine Flugzeitmessung eines Heizimpulses verwendet wird. Dazu wird die Temperaturveränderung in einem kleinen Abstand vom Heizelement gemessen und zur Temperaturvariation des Heizelements korreliert. Zudem wird der Druck des Fluids gemessen und daraus die Wärmekapazität und die Zusammensetzung des Fluids bestimmt.

Die WO 2004/065914 A1 beschreibt einen Gaszähler, bei dem mehrere Korrekturfaktoren erfasst werden, um die Menge an bezogenem Energieinhalt des Gases zu erfassen.

Die WO 2001/18500 A1 beschreibt ein Verfahren zum Messen des Massenflusses eines Fluids, bei dem das Fluid über einen ersten Temperatursensor, ein Heizelement und einen zweiten Temperatursensor geführt und der Massenfluss aus mindestens einem Temperatursignal der Temperatursensoren ermittelt wird. Zur Erhöhung der Messgenauigkeit wird mindestens eine stoffspezifische Kenngröße zur Charakterisierung des Wärmeübergangsverhaltens des Fluids gemessen wird und die Massenflussmessung mit Hilfe der stoffspezifischen Kenngröße korrigiert.

Aus der DE 10 2012 019 657 ist bekannt, dass die Wärmeleitfähigkeit und die Temperaturleitfähigkeit eines gasförmigen Fluids dazu verwendet werden können, das vermessene Fluid zu charakterisieren. Es hat sich jedoch herausgestellt, dass die Messunsicherheit bei derartigen Verfahren vergleichsweise hoch ist. Der Grund dafür ist, dass sich die Temperaturleitfähigkeit und Wärmeleitfähigkeit kaum ändern, auch wenn sich die Zusammensetzung des Fluids deutlich ändert. Das ist besonders bei brennbaren Gasen, wie beispielsweise bei Erdgas der Fall.

Der Erfindung liegt die Aufgabe zugrunde, die Bestimmung des die Zusammensetzung des gasförmigen Fluids charakterisierenden Parameters, insbesondere eines den Energieinhalt des Fluids charakterisierenden Energieinhalts-Parameters, zu verbessern.

Die Erfindung löst das Problem durch einen Gaszähler mit den Merkmalen von Anspruch 1.

Vorteilhaft an der Erfindung ist, dass der die Zusammensetzung des gasförmigen Fluids charakterisierende Parameter mit einer deutlich geringeren Messunsicherheit bestimmt werden kann.

Vorteilhaft ist zudem, dass die geringere Messunsicherheit mit keinem oder einem nur unwesentlich höheren apparativen Aufwand einhergeht. Es ist möglich, dass die Wärmequelle und das Thermometer auf einem integrierten Schaltkreis (Chip) integriert sind. Insbesondere ist es möglich, dass die Wärmequelle und das zumindest eine Thermometer durch Herausätzen aus einem Siliziumsubstrat hergestellt und/oder einstückig mit dem verbleibenden Teil des Substrats verbunden sind. Die Herstellung eines derartigen Gas-Zusammensetzungssensors ist kostengünstig in einem Massenverfahren möglich.

Der Erfindung liegt unter anderem die Erkenntnis zugrunde, das jeder Festkörper, insbesondere also die Wärmequelle und das Thermometer, von einem ballistischen Bereich umgeben sind, in dem die Wärmeleitung nicht durch Diffusion, sondern nur durch ballistischen Transport zutreffend beschreibbar ist. Der Grund dafür ist, dass in unmittelbarer Umgebung mit der Oberfläche des Festkörpers die Bewegungsmöglichkeiten der Gasteilchen in eine Richtung (nämlich auf die Wand des Festkörpers hin) eingeschränkt sind. Ein diffuserer Bereich beginnt ungefähr bei einem Abstand, der größer ist als das Zehnfache der mittleren freien Weglänge. Ist zwischen der Wärmequelle und dem Thermometer kein weiterer Festkörper angeordnet, was eine bevorzugte Ausführungsform darstellt, so beträgt der Abstand zwischen der Wärmequelle und dem Thermometer folglich höchstens das 500-fache der mittleren freien Weglänge.

Bei einem so kleinen Abstand zwischen der Wärmequelle und dem Thermometer führt die Auswertung der zeitabhängig gemessenen Temperatur, beispielsweise auf Basis der Laufzeit des Temperaturimpulses von der Wärmequelle zum Thermometer, unter Verwendung einer Formel auf Basis der Fourier-Gleichung zu einer systematischen Messabweichung.

Die systematische Messabweichung hängt von der Atom- oder Molekülmasse ab. Es ist möglich, dass die systematische Messabweichung zudem von anderen Parametern abhängt, die weiter unten erläutert werden. Aus diesem Grund unterscheiden sich die Laufzeiten der Wärmeimpulse für unterschiedliche Fluide, die sich in ihren jeweiligen Temperaturleitfähigkeiten kaum unterscheiden. Überraschend dabei ist, dass der ballistische Bereich bereits dann einen signifikanten Einfluss hat, wenn er vergleichsweise klein ist gegenüber dem diffusiven Bereich.

An der Wand der Wärmequelle und des Thermometers kommt es zu einer unvollständigen Temperaturakkomodation, die sich als Differenz zwischen Gas- und Wandtemperatur an einer Wandoberfläche bemerkbar macht. In Analogie zur Impulsakkomodation wird die unvollständige Temperaturakkomodation durch einen Temperatur- oder Energieakkomodationskoeffizienten $\alpha_E$ beschrieben:

$$\alpha_E = \frac{T_e - T_r}{T_e - T_W}.$$

Die Extremwerte sind: $\alpha_E = 1 \rightarrow T_r = T_W$ vollständige thermische Anpassung und $\alpha_E = 0 \rightarrow T_r = T_e$ keine Anpassung an Wandzustand ($q = 0$). Der Wärmestrom q lässt sich dann beschreiben durch

$$q = \frac{k_q}{6} \varrho \tilde{c}_0 c_v \frac{\alpha_E}{2 - \alpha_E} \ (T_{W2} - T_{W1}). \tag{1}$$

Darin sind:

$\varrho$    Massendichte

$\tilde{c}_0$   $\tilde{c}_0 = \sqrt{3RT}$ (R: Gaskonstante, T: Temperatur)

$\tilde{c}_v$ spezifische Wärmekapazität (bei konstantem Volumen)

$k_q$ Wärmeleitfähigkeit Korrekturfaktor (i. Allgem. $k_q \approx 1$)

$\alpha_E$: Temperatur-Akkomodationskoeffizient

$T_{W1}, T_{W2}$ die Wandtemperaturen.

Der Akkomodationskoeffizient $\alpha_E$ ist abhängig von verschiedenen Parametern, wie Knudsen-Zahl, Gaszustand, Wandmaterial und Oberflächenstruktur. Der Vorfaktor vor dem Klammerausdruck mit der Differenz der Temperaturen unterscheidet sich von dem Vorfaktor bei diffusivem Wärmetransport.

Im Rahmen der vorliegenden Beschreibung wird unter dem die Zusammensetzung des gasförmigen Fluids charakterisierenden Parameter insbesondere ein den Energieinhalt des Fluids charakterisierender Energieinhalts-Parameter verstanden. Bei dem Parameter kann es sich beispielsweise um den Brennwert, Heizwert oder einen sonstigen den Energieinhalt des Fluids beschreibenden Parameter handeln.

Bei dem Parameter kann es sich um eine einkomponentige oder skalare Größe oder um eine mehrkomponentige oder vektorielle Größe handeln, beispielsweise den Vektor, dessen Einträge zwei, drei oder mehr Konzentrationen von Gasbestandteilen des Fluids sind.

Der den Energieinhalt des Fluids charakterisierende Energieinhalts-Parameter kann beispielsweise die Beschaffenheit und/oder die Klassifikation eines Erdgases sein. Derartige Klassifikationen geben an, um welche Art von Erdgas es sich handelt. Erdgas wird in verschiedenen Qualitäten gehandelt, die sich hinsichtlich Brennwert unterscheiden. Der Brennwert des Erdgases einer vorgegebenen Klassifikation ändert sich kaum mit der Zeit, hingegen unterscheiden sich die Brennwerte der Erdgase verschiedener Klassifikationen voneinander. Um das gelieferte Erdgas nach seinem Energieinhalt abrechnen zu können, ist es daher ausreichend, die Klassifikation des Erdgases zu kennen.

Die Klassifikation des Erdgases ist insbesondere eine Herkunfts-Klassifikation. Die Zusammensetzung von Erdgas unterscheidet sich je nach Fördergebiet. Auch der Heizwert und der Brennwert unterscheiden sich je nach Fördergebiet.

Grundsätzlich hängen die thermischen Transportgrößen, also die Wärme- und die Temperaturleitfähigkeit, nicht vom Heizwert und/oder dem Brennwert ab. Das ist bereits daran erkennbar, dass auch Gase ohne Brennwert, wie Kohlendioxid, eine Wärme- und eine Temperaturleitfähigkeit haben. Gasgemische, beispielsweise aus Methan, Kohlendioxid, Propan und Wasserstoff unterschiedliche Brennwerte bei gleicher Temperaturleitfähigkeit haben. In Bezug auf den erfindungsgemäßen Gaszähler ist es eine relevante Einsicht des Erfinders, dass für die üblichen gehandelten Erdgasarten aus der Temperaturleitfähigkeit auf die Herkunft des Erdgases und damit auf den Energieinhalts-Parameter, insbesondere den Heizwert und/oder den Brennwert, geschlossen werden kann. Erst auf Basis dieser Erkenntnis ist es möglich, von einer thermischen Transportgröße auf den Energieinhalt von Erdgas zu schließen.

Unter dem impulsförmigen Erwärmen wird insbesondere ein Erwärmen verstanden, bei dem die Impulsdauer höchstens 500 Mikrosekunden, vorzugsweise höchstens 250 Mikrosekunden, beträgt. Gut geeignet ist eine Impulsdauer zwischen 80 und 120 Mikrosekunden. Vorzugsweise ist die Impulsdauer größer als 10 Mikrosekunden.

Unter dem zeitabhängigen Messen wird insbesondere verstanden, dass mehrere Messwerte für die Temperaturen in zeitlichen Abständen aufgenommen werden. Es ist möglich und stellt eine bevorzugte Ausführungsform dar, dass zu jedem Temperaturmesswert die Zeit erfasst und mit dem Messwert für die Temperatur verknüpft wird. Es ist aber auch möglich, dass mehrere Temperatur-Messwerte aufgenommen werden und daraus beispielsweise der Maximalwert ermittelt wird. Insbesondere führt das zeitabhängige Messen der Temperatur also zu einem Temperaturverlauf, wobei der Parameter aus dem Temperaturverlauf ermittelt wird.

Unter dem Ermitteln des Parameters aus der gemessenen Temperatur wird insbesondere verstanden, dass der Parameter in Abhängigkeit von zumindest einem Temperatur-Messwert ermittelt wird. Das kann beispielsweise dadurch geschehen, dass die Temperaturen oder ein aus den Temperaturen ermittelter Mess-Parameter mit einem in Versuchen ermittelten Kennfeld verglichen werden.

Vorzugsweise ist die Wärmequelle länglich ausgebildet, das heißt, dass sie in einer Raumdimension zumindest zehnmal so lang ist wie in den beiden senkrecht dazu verlaufenden anderen Raumdimensionen.

Vorzugsweise umfasst das Ermitteln des Parameters die Schritte eines Messens zumindest einer Laufzeitdifferenz zwischen dem Zeitpunkt des impulsförmigen Erwärmens und dem Zeitpunkt, zu dem eine vom Thermometer gemessene Temperatur ihr Maximum hat, sowie eines Bestimmens des Parameters aus der zumindest einen Laufzeitdifferenz. Es sei darauf hingewiesen, dass es möglich, nicht aber notwendig ist, dass die Temperatur in einer Temperatureinheit gemessen wird. Insbesondere ist es auch möglich, dass ein Messwert, beispielsweise ein elektrischer Messwert, aufgenommen wird, der eindeutig mit der Temperatur zusammenhängt. Zur Berechnung der Laufzeitdifferenz ist es dann ausreichend, den Zeitpunkt zu bestimmen, zu dem dieser Messwert sein Maximum durchläuft.

Gemäß einer bevorzugten Ausführungsform ist das Fluid Erdgas und der der Parameter B ist eine Klassifikation, die den Förderort oder das Fördergebiet des Erdgases kodiert. Für Erdgas ist es wünschenswert, den Verbrauch als brennwertgewichteten Durchfluss direkt in der Dimension Arbeit anzugeben, also als Produkt aus verbrauchtem Gasvolumen und dem jeweiligen Heiz- oder Brennwert. Die Bestimmung des Heiz- oder Brennwerts war bislang aufwändig. Dadurch, dass, wie weiter unten näher erläutert, aus der thermischen Transportgröße auf den Heiz- oder Brennwerts geschlossen werden kann, wenn die zusätzliche Information vorliegt, dass es sich bei dem Gas um Erdgas handelt, kann der Verbrauch deutlich einfacher in Form der Arbeit, also in Kilowattstunden oder Joule, angegeben werden.

Insbesondere umfasst das Ermitteln des Parameters B anhand der zumindest einen gemessenen Temperatur, insbesondere zumindest drei gemessenen Temperaturen, ein Auslesen und/oder Interpolieren eines Kennfelds ist, das den Laufzeitdifferenzen den Parameter B zuordnet. Dass dieses Auslesen überhaupt funktioniert, liegt darin begründet, dass einerseits nur wenige Parameter Klassifikationen für Erdgas existieren, und andererseits, dass durch den geringen Abstand eines Thermometers von maximal 60 $\mu$m und den deutlich größeren Abstand des zweiten und ggf. dritten Thermometers die Eigenschaften des Gases sowohl bei nicht-ballistischem als auch bei ballistischem Transports erfasst werden.

Vorzugsweise sind die Wärmequelle und das Thermometer so relativ zueinander angeordnet sind, dass die erste Laufzeitdifferenz für das Thermometer, das der Wärmequelle am nächsten liegt, kleiner ist als das Fünffache einer Impulsdauer des impulsförmigen Erwärmens. Es wäre anzunehmen gewesen, dass ein so geringer Abstand zwischen Wärmequelle und Thermometer problematisch ist, weil dann die Näherung des Wärmeimpulses als deltafunktionsförmig keine gute Näherung mehr ist. Es hat sich aber gezeigt, dass so Gase unterschiedlicher Temperaturleitfähigkeiten besonders gut unterscheidbar sind.

Vorzugsweise umfasst das Verfahren die Schritte (a) Messen einer zweiten Laufzeit zwischen dem Zeitpunkt des impulsförmigen Erwärmens in dem Zeitpunkt, zu dem eine am zweiten Thermometer anliegende Temperatur ihr Maximum hat, wobei das zweite Thermometer in einem zweiten Abstand von der Wärmequelle angeordnet ist, der größer ist als ein erster Abstand, in dem das erste Thermometer von der Wärmequelle angeordnet ist, und (b) Ermitteln des Parameters aus den Laufzeitdifferenzen. Günstig ist es, wenn der zweite Abstand größer ist als das 300-fache, insbesondere das 500-fache, der mittleren freien Weglänge der Gasteilchen des Fluids. In diesem Fall ist der Einfluss des ballistischen Bereichs auf die Messung mit dem zweiten Thermometer deutlich kleiner als auf die Messung mit dem ersten Thermometer, sodass die Kombination der beiden Messungen zu einer genaueren Bestimmung des Parameters führt.

Vorzugsweise beträgt der zweite Abstand zumindest das Doppelte, insbesondere zumindest das Dreifache, des ersten Abstands. Besonders bevorzugt beträgt der zweite Abstand zumindest das Fünffache des ersten Abstands.

Vorzugsweise wird eine dritte Laufzeit zwischen der Wärmequelle und einem dritten Thermometer bestimmt, wobei der dritte Abstand des dritten Thermometers von der Wärmequelle größer ist als der zweite Abstand.

Die Wärmequelle ist vorzugsweise länglich. Hierunter ist insbesondere zu verstehen, dass die Wärmequelle als linienförmig angesehen werden kann und eine systematische Messunsicherheit, die durch diese Annahme bedingt ist, kleiner ist als 5%. Vorzugsweise ist die Wärmequelle als freitragender Steg ausgebildet..

Vorzugsweise umfasst das Verfahren die Schritte des Messens der Wärmeleitfähigkeit des Fluids und des Bestimmens der Zusammensetzung des Fluids aus den Laufzeitdifferenzen und der Wärmeleitfähigkeit. Wie die Wärmeleitfähigkeit gemessen werden kann, ist in den Patentanmeldungen DE 10 2014 010 939, DE 10 2012 019 657 und DE 10 2011 121 213 beschrieben und wird hier daher nicht weiter ausgeführt.

Vorzugsweise ist die Steuerung eingerichtet zum automatischen Ermitteln eines Durchflusses an gasförmigem Fluid und Ermitteln des die Zusammensetzung eines gasförmigen Fluids charakterisierenden Parameters in Form des Brennwerts.

Die Steuerung ist zudem eingerichtet zum automatischen Berechnen des Energieinhalts des durchgeflossenen Fluids, beispielsweise in Form des Produkts aus Durchfluss und Brennwert. Auf diese Weise kann auch bei schwankender Gaszusammensetzung stets nach bezogener Energie abgerechnet werden. Der Durchfluss ist insbesondere ein Massendurchfluss oder ein Volumendurchfluss.

Günstig ist es, dass das Fluid bei der Messung der zumindest einen Laufzeitdifferenz relativ zur Wärmequelle ruht. Beispielsweise ist der Gas-Zusammensetzungssensor in einer Abzweigleitung der Durchleitung angeordnet, in der das gleiche Fluid vorliegt wie in der Durchleitung und in der das Fluid eine so geringe Geschwindigkeit hat, dass es als ruhend angesehen werden kann.

Daher besitzt der Gaszähler (a) eine Einströmöffnung, (b) eine Ausströmöffnung und (c) eine strömungsgeschwindigkeitsberuhigten Zone, die in Fluidverbindung mit der Einströmöffnung und der Ausströmöffnung steht und so ausgebildet ist, dass eine mittlere Strömungsgeschwindigkeit in der strömungsgeschwindigkeitsberuhigten Zone höchstens ein Zehntel, vorzugsweise höchstens ein Zwanzigstel, einer mittleren Strömungsgeschwindigkeit durch die Einströmöffnung beträgt, wobei der Gas-Zusammensetzungssensors in der strömungsgeschwindigkeitsberuhigten Zone angeordnet ist. Die mittlere Strömungsgeschwindigkeit ist der Quotient aus dem Durchfluss als Zähler und der Querschnittsfläche als Nenner.

Gemäß einer bevorzugten Ausführungsform besitzt der Gaszähler einen Geschwindigkeitssensor zum Messen der Geschwindigkeit des Fluids und/oder des Durchflusses. Ein solcher Sensor ist in der DE 10 2012 019 657 oder der DE 10 2014 010 939 beschrieben. Aus der Geschwindigkeit des Fluids und/oder des Durchflusses einerseits und dem den Heizwert charakterisierenden Parameter wird von der Steuerung der Energieinhalt des durchgeflossenen Fluids berechnet.

Im Folgenden wird die Erfindung anhand der beigefügten Zeichnungen näher erläutert. Dabei zeigt

Figur 1    in Teilfigur 1a eine schematische Ansicht eines erfindungsgemäßen Gaszählers mit einem Gas-Zusammensetzungssensor und in Teilfigur 1b eine schematische Ansicht eines erfindungsgemäßen Erdgasnetzwerks, das den Gaszähler umfasst,

Figur 2    eine schematische Totalansicht des Gas-Zusammensetzungssensors, und

Figur 3    ein Diagramm, in dem die systematische Messabweichung in Abhängigkeit des Abstandes zwischen der Wärmequelle und dem Thermometer aufgezeichnet ist, und

Figur 4    ein Diagramm, in dem ein Parameter B in Form einer Klassifikation für Erdgas und der zugehörige Brennwert sowie der Wobbe-Index $W$ angegeben sind.

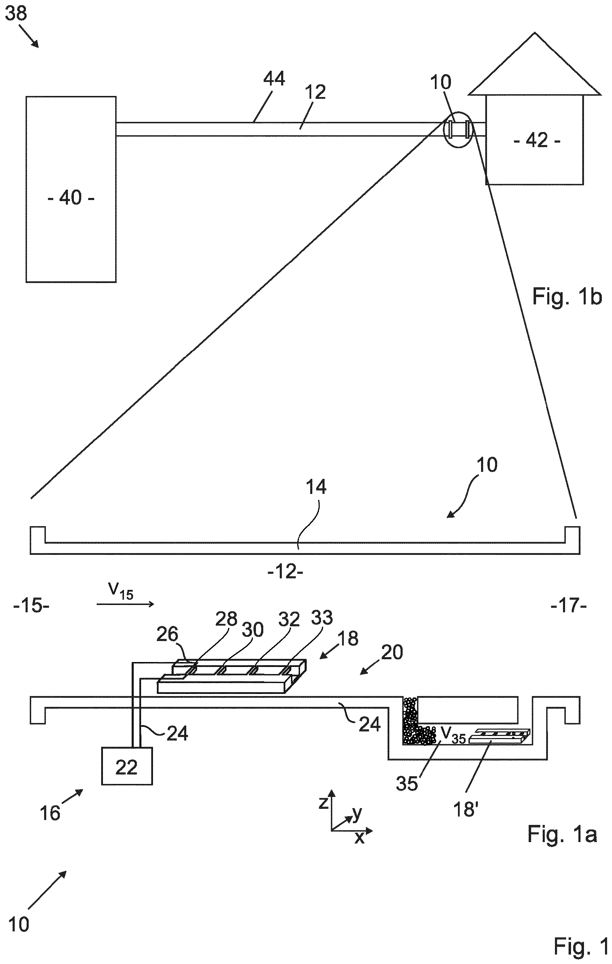

Figur 1a zeigt einen schematischen Querschnitt durch einen erfindungsgemäßen Gaszähler 10 zum Messen des energieinhaltgewichteten Durchflusses $D_{BW}$ eines gasförmigen Fluids 12, der eine Durchleitung 14 und ein Gas-Zusammensetzungssensor 16 aufweist. Der energieinhaltgewichtete Durchfluss $D_{BW}$ ist das Produkt $D_{BW} = D \cdot H$ aus dem Durchfluss $D = \dot{V}$, der beispielsweise in Liter pro Sekunde angegeben wird, und dem Heizwert $H$ oder alternativ dem Brennwert in Joule pro Liter. Es ergibt sich damit für den energieinhaltgewichteten Durchfluss $D_{BW}$ die Dimension einer Energie pro Zeiteinheit. Bei dem Fluid 12 handelt es sich vorzugsweise um Erdgas. Alternativ oder zusätzlich erfasst der Gaszähler 10 die brennwertgewichtete Durchflussmenge, beispielsweise in Joule oder Kilowattstunden.

Der Gaszähler 10 besitzt eine Einströmöffnung 15, eine Ausströmöffnung 17 und ein Messelement 18, das beispielsweise dicht an einer Innenoberfläche 20 der Durchleitung 14 angeordnet ist. Das Messelement 18 ist mit einer Steuerung 22, die selbsttätig das Messelement 18 ansteuert, mittels einer Leitung 24 verbunden.

Es ist zu erkennen, dass das Messelement 18 ein Substrat 26 aufweist, aus dem eine längliche Wärmequelle 28 durch Ätzen freigelegt wurde. Auf diese Weise bildet die Wärmequelle einen Steg. Auf die gleiche Weise wurden aus dem Substrat 26 ein erstes Thermometer 30, ein zweites Thermometer 32 und ein drittes Thermometer 33 herausgearbeitet.

Um die Gaszusammensetzung auch dann hochgenau messen zu können, wenn sich das Fluid 12 bewegt, kann das Messelement wie mit dem Bezugszeichen 18' gekennzeichnet in einer strömungsgeschwindigkeitsberuhigten Zone, beispielsweise einer Abzweigleitung 35, angeordnet sein. In der strömungsgeschwindigkeitsberuhigten Zone hat das Fluid eine Strömungsgeschwindigkeit v, die in guter Näherung als null angesehen werden kann. Meist ist die Strömungsgeschwindigkeit v in der strömungsgeschwindigkeitsberuhigten Zone deutlich kleiner als in der Durchleitung 14.

Ist ein solches Messelement 18' vorhanden, kann das Messelement 18 auch zum Messen der Geschwindigkeit v verwendet werden. Das entsprechende Verfahren ist in der DE 10 2012 019 657 oder der DE 10 2014 010 939 beschrieben.

Ist das Messelement 18 wie gezeigt in der Durchleitung 14 angeordnet, so wird die Gaszusammensetzung vorzugsweise dann ermittelt, wenn die Strömungsgeschwindigkeit v hinreichend klein ist. Dazu kann ebenfalls ein in der DE 10 2012 019 657 oder der DE 10 2014 010 939 beschriebenes Verfahren eingesetzt werden.

Figur 1b zeigt, dass der Gaszähler 10 Teil eines Erdgasnetzes 38 ist, das eine Erdgasquelle 40, beispielsweise eine Pipeline aus einem Fördergebiet oder einen Kavernenspeicher, und einen Erdgasverbraucher 42, insbesondere einen Privathaushalt, sowie eine Erdgasleitung 44 aufweist. Die Erdgasleitung 44 umfasst den Gaszähler 10, der als Erdgaszähler ausgebildet ist. Der Erdgaszähler 10 ist Teil der Erdgasleitung 44. Es ist möglich und stellt eine bevorzugte Ausführungsform dar, dass der Erdgaszähler zum elektronischen Übermitteln des gemessenen brennwertgewichteten Durchflusses an einen räumlich beabstandeten Rechner, beispielsweise einer Abrechnungsstelle.

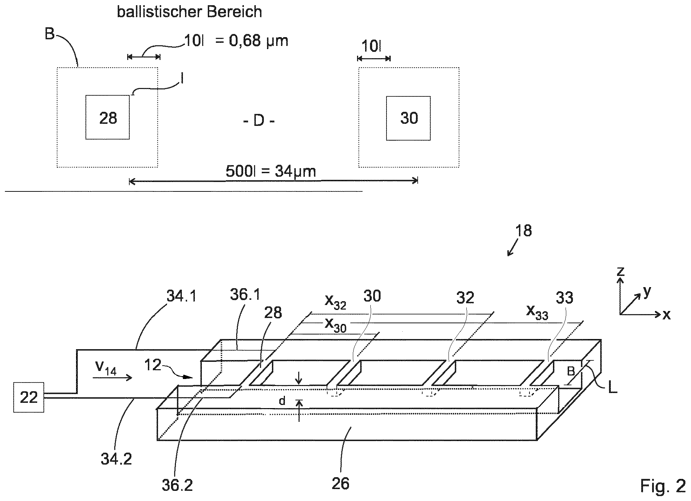

Figur 2 zeigt in der Teilfigur 2a das Messelement 18 in einer vergrößerten Ansicht. Es ist zu erkennen, dass die Steuerung 22 die Wärmequelle 28 elektrisch über Leitungen 24.1, 24.2 und daran jeweils anschließende Leiterbahnen 36.1, 36.2 kontaktiert. Die Wärmequelle 28 hat eine Länge L in y-Richtung, die deutlich größer ist als deren Breite und Dicke, also deren Ausdehnung in x-Richtung und z-Richtung.

Das erste Thermometer 30 ist auf die gleiche Weise kontaktiert, die Anschlüsse an die Steuerung 22 sind der Übersichtlichkeit halber aber nicht mit eingezeichnet. Das erste Thermometer 30 ist in Form einer Wheatstone-Brücke verschaltet, sodass sein elektrischer Widerstand zumindest weitgehend stromfrei gemessen werden kann. Die Abhängigkeit des elektrischen Widerstands von der Temperatur $T_{30}$ ist bekannt, sodass aus dem Widerstand auf die Temperatur $T_{30}$ geschlossen werden kann. Ein erster Abstand $x_{30}$ des ersten Thermometers 30 von der Wärmequelle 28 beträgt im vorliegendem Fall 40 $\mu m$. Ein zweiter Abstand $x_{32}$ zwischen dem zweiten Thermometer 32 und der Wärmequelle 28 beträgt $x_{32} = 100$ $\mu m$.

Wird der Gaszähler 10 wie gemäß einer bevorzugten Ausführungsform vorgesehen bei Normaldruck von $p = 1013\ hPa$ und einer Temperatur von $T = 23°C$ mit Methan betrieben, so beträgt die mittlere freie Weglänge $\lambda$ ungefähr $\lambda = 68\ nm$. Wie in dem Teilbild 2b oben schematisch eingezeichnet ist, kann ein ballistischer Bereich B als die Hülle im Abstand von $10\lambda$ um die Wärmequelle 28 sowie die Thermometer 30, 32 angesehen werden. Im ballistischen Bereich B kann die Wärmeausbreitung nicht durch Wärmeleitung nach den Fourier-Gleichungen beschrieben werden, sondern anhand von Gleichung (1). Die Ausdehnung des ballistischen Bereichs B zwischen der Wärmequelle 28 und dem ersten Thermometer 30 beträgt damit $2 \times 10\lambda = 1,36\ \mu m$. Das 50-fache der Ausdehnung des ballistischen Bereichs beträgt damit $86\mu m$, was kleiner ist als der erste Abstand $x_{30}$. Ganz allgemein ist der erste Abstand bevorzugt größer als das Doppelte der mittleren freien Weglänge. Vorzugsweise ist der erste Abstand größer als 3 $\mu m$.

Der Bereich D außerhalb des ballistischen Bereichs B ist der diffusive Bereich, in dem die Wärmeausbreitung durch die Fourier-Gleichung beschrieben werden kann.

Im Betrieb des erfindungsgemäßen Gaszählers gibt die Steuerung 22 einen kurzen Stromimpuls ab, sodass die Wärmequelle 28 einen Wärmeimpuls abgibt, dessen Impulsdauer beispielsweise höchstens $\tau = 100$ Mikrosekunden beträgt. Die Impulsdauer $\tau$ ist, wenn der Stromimpuls nicht streng rechteckförmig ist, diejenige Zeit, in der 95 % der der Wärme des Wärmeimpulses abgegeben wurde. Simultan wird eine erste Temperatur $T_{30}$ vom ersten Thermometer 30 in Abhängigkeit von der Zeit $t$ gemessen. Aus dem so erhaltenen Temperaturverlauf $T_{30}(t)$ wird eine erste Laufzeit $\Delta t_1$ ermittelt, die nach dem Temperaturimpuls vergeht, bis das Temperaturmaximum das erste Thermometer 30 passiert hat. Auf die gleiche Weise werden in der vorliegenden Ausführungsform eine zweite Laufzeit $\Delta t_2$ aus den Temperatur-Messdaten $T_{32}(t)$, die mittels des zweiten Thermometers 32 zeitabhängig von der Steuerung 22 aufgenommen wird, und eine dritte Laufzeit $\Delta t_3$ aus den Temperatur-Messdaten $T_{33}(t)$, die mittels des dritten Thermometers 33 zeitabhängig von der Steuerung 22 aufgenommen wird, ermittelt.

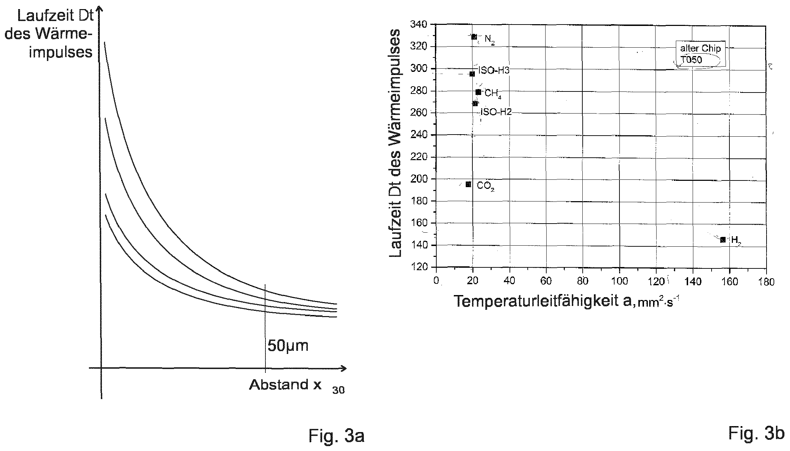

Figur 3a zeigt ein Diagramm, in dem die Laufzeit $\Delta t$ des Wärmeimpulses in Abhängigkeit von dem ersten Abstand $x_{30}$ (vgl. Figur 2a) für verschiedene Fluide aufgetragen ist. Es ist zu erkennen, dass die Unterschiede mit abnehmendem ersten Abstand $x_{30}$ größer werden.

Figur 3b zeigt Laufzeiten $\Delta t$ und Temperaturleitfähigkeiten a für verschiedene Fluide.

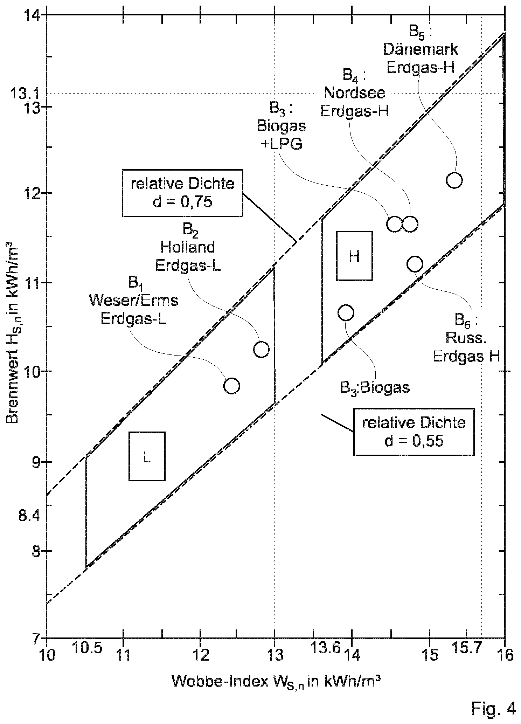

Figur 4 zeigt ein Diagramm, in dem ein Parameter B in Form einer Klassifikation für Erdgas und der zugehörige Brennwert sowie der Wobbe-Index $W$ angegeben sind. Der Parameter B ist Teil einer Menge $B_i$ mit $i = 1, 2, ..., N$, wobei N vorzugsweise höchstens 10, insbesondere höchstens 8 ist. Da die Menge der $B_i$ relativ klein ist, kann aus der ersten Laufzeit $\Delta t_1$ oder dem Vektor $(\Delta t_1, \Delta t_2)$ aus der ersten und der zweiten Laufzeit mit hoher Sicherheit der Parameter B identifiziert werden. Anhand des Diagramms gemäß Figur 4 kann daraus der Heizwert, der Brennwert und/oder der Wobbe-Index ermittelt werden.

**Bezugszeichen**

| | | | |
|---|---|---|---|
| 10 | Gaszähler | | |
| 12 | Fluid, Erdgas | $\lambda_w$ | mittlere freie Weglänge |
| 14 | Durchleitung | $\alpha$ | Temperaturleitfähigkeit |
| 15 | Einströmöffnung | B | Parameter |
| 16 | Gas-Zusammensetzungssensor | $D$ (= V) | Durchfluss |
| 17 | Ausströmöffnung | $D_{BW}$ | brennwertgewichteter Durch-fluss |
| 18 | Messelement | | |
| | | $H$ | Heizwert |
| 20 | Innenoberfläche | L | Länge |
| 22 | Steuerung | p | Druck |
| 24 | Leitung | | |
| 26 | Substrat | $\tau$ | Impulsdauer |
| 28 | Wärmequelle | t | Zeit |
| 30 | erstes Thermometer | $T_{30}$ | erste Temperatur |
| | | $T_{32}$ | zweite Temperatur |
| 32 | zweites Thermometer | $x_{30}$ | erster Abstand |
| 33 | drittes Thermometer | $x_{32}$ | zweiter Abstand |
| 34 | Leitung | | |
| 35 | strömungsgeschwindigkeitsberuhigte Zone, Abzweigleitung | $\Delta t_1$ | erste Laufzeit |
| | | $\Delta t_2$ | zweite Laufzeit |
| 36 | Leiterbahn | $\Delta t_3$ | dritte Laufzeit |
| 38 | Erdgasnetz | V | Strömungsgeschwindigkeit |
| 40 | Erdgasquelle | $W$ | Wärmepfad |
| 42 | Erdgasverbraucher | | |
| 44 | Erdgasleitung | | |

**Patentansprüche**

1. Gaszähler mit

   (a) einer Durchleitung (14) und
   (b) einem Gas-Zusammensetzungssensor (16) zum Bestimmen eines die Zusammensetzung eines gasförmigen Fluids (12) charakterisierenden Parameters (B), mit

      (i) einer Wärmequelle (28) zum impulsförmigen Erwärmen des zu vermessenden Fluids (12),
      (ii) zumindest einem Thermometer (30, 32, 33) zum zeitabhängigen Messen einer Temperatur des Fluids (12) und
      (iii) einer Steuerung (22), die ausgebildet ist zum automatischen

         - impulsförmigen Erwärmen des zu vermessenden Fluids (12) mittels der Wärmequelle (28),
         - zeitabhängigen Messen der Temperatur ($T_{30}$, $T_{32}$, $T_{33}$) des Fluids (12) mittels des zumindest einen Thermometers (30, 32, 33) und
         - Ermitteln des Parameters (B) anhand der zumindest einen gemessenen Temperatur ($T_{30}$, $T_{32}$, $T_{33}$),

      (iv) wobei ein Abstand ($x_{30}$) zumindest eines der Thermometer (30, 32) von der Wärmequelle (28) maximal 100 μm beträgt und
      (v) wobei die Steuerung des Gas-Zusammensetzungssensors (16) eingerichtet ist zum automatischen

         - Ermitteln eines Durchflusses des gasförmigen Fluids (12),
         - Ermitteln des die Zusammensetzung eines gasförmigen Fluids (12) charakterisierenden Parameters (B) des Fluids (12) in Form eines Energieinhalts-Parameters (B) und
         - Berechnen des Produkts aus Durchfluss (D) und Energieinhalts-Parameter (B).

(c) einer Einströmöffnung (15) und
(d) einer Ausströmöffnung (17) **gekennzeichnet durch**
(e) eine strömungsgeschwindigkeitsberuhigte Zone (35), die

- in Fluidverbindung mit der Einströmöffnung (15) und der Ausströmöffnung (17) steht und
- so ausgebildet ist, dass eine mittlere Strömungsgeschwindigkeit ($v_{35}$) in der strömungsgeschwindigkeitsberuhigten Zone (35) höchstens ein Zehntel einer mittleren Strömungsgeschwindigkeit durch die Einströmöffnung ($v_{15}$) beträgt,

(f) wobei der Gas-Zusammensetzungssensor (16) in der strömungsgeschwindigkeitsberuhigten Zone (35) angeordnet ist.

2. Gaszähler nach einem der vorstehenden Ansprüche, **dadurch gekennzeichnet, dass** die Steuerung (22) eingerichtet ist zum Durchführen eines Verfahrens, bei dem das Ermitteln des Parameters die folgenden Schritte aufweist:

(a) Messen zumindest einer Laufzeitdifferenz ($\Delta t_1$, $\Delta t_2$, $\Delta t_3$) zwischen dem Zeitpunkt des impulsförmigen Erwärmens und
dem Zeitpunkt, zu dem eine vom Thermometer (30, 32) gemessene Temperatur ($T_{30}$, $T_{32}$) ihr Maximum hat, und
(b) aus der zumindest einen Laufzeitdifferenz ($\Delta t_1$, $\Delta t_2$, $\Delta t_3$) Bestimmen des Parameters (B).

3. Gaszähler nach einem der vorstehenden Ansprüche, **dadurch gekennzeichnet, dass**

(a) er ein Erdgaszähler ist,
(b) der Parameter (B) eine Klassifikation, die den Förderort des Erdgases kodiert, ist und
(c) das Ermitteln des Parameters (B) anhand der zumindest einen gemessenen Temperatur ($T_{30}$, $T_{32}$, $T_{33}$) ein Auslesen und/oder Interpolieren eines Kennfelds ist, das der zumindest einen Laufzeitdifferenz ($\Delta t_1$, $\Delta t_2$, $\Delta t_3$) den Parameter (B) zuordnet.

4. Gaszähler Gas-Zusammensetzungssensor (16) nach einem der vorstehenden Ansprüche , **dadurch gekennzeichnet, dass**
das Ermitteln des Parameters (B) anhand der zumindest einen gemessenen Temperatur ($T_{30}$, T32, T33) ein Auslesen und/oder Interpolieren eines Kennfelds ist, das der zumindest einen Laufzeitdifferenz ($\Delta t_1$, $\Delta t_2$, $\Delta t_3$) den Parameter (B) zuordnet.

5. Erdgasnetzwerk mit

(i) einer Erdgasquelle (40),
(ii) einem Erdgasverbraucher (42) und
(iii) einer Erdgasleitung (44) zum Fördern von Erdgas (12) von der Erdgasquelle (40) zum Erdgasverbraucher (42),

**gekennzeichnet durch**
(iv) einen Erdgaszähler (10) gemäß einem der vorstehenden Ansprüche, der Teil der Erdgasleitung (44) ist.

6. Verwendung eines Gaszählers nach einem der vorstehenden Ansprüche 1 bis 4 zur Bestimmung eines brennwertgewichteten Durchflusses ($D_{BW}$) von Erdgas.

**Claims**

1. A gas meter with

(a) a through-line (14) and
(b) a gas composition sensor (16) for determining one of the parameters (B) that characterises the composition of a gaseous fluid (12), with

(i) a heat source (28) for pulsed heating of the fluid to be measured (12),
(ii) at least one thermometer (30, 32, 33) for time-dependent measurement of a temperature of the fluid (12) and

(iii) a control unit (22) that is designed for the automatic

- pulsed heating of the fluid (12) to be measured by means of the heat source (28),
- time-dependent measurement of the temperature ($T_{30}$, $T32$, $T33$) of the fluid (12) by means of the at least one thermometer (30, 32, 33) and
- detection of the parameter (B) using the at least one measured temperature ($T_{30}$, $T32$, $T33$),

(iv) wherein a distance ($x_{30}$) of at least one of the thermometers (30, 32) from the heat source (28) is a maximum of 100 $\mu$m and
(v) wherein the control unit of the gas composition sensor (16) is configured to automatically

- detect a through-flow of the gaseous fluid (12),
- detect the parameter (B) of the fluid (12) that characterises the composition of a gaseous fluid (12) in the form of an energy content parameter (B) and
- calculate the product of through-flow (D) and energy-content parameter (B).

(c) an inlet opening (15) and
(d) an outlet opening (17)

**characterised by**

(e) a flow velocity reduction zone (35) which

- is in fluid connection to the inlet opening (15) and the outlet opening (17) and
- is designed in such a way that an average flow velocity ($v_{35}$) in the flow velocity reduction zone (35) is at most one tenth of an average flow velocity through the inlet opening ($v_{15}$),

(f) wherein the gas composition sensor (16) is arranged in the flow velocity reduction zone (35).

2. The gas meter according to one of the preceding claims, **characterised in that** the control unit (22) is configured to conduct a method in which the detection of the parameter contains the following steps:

(a) measuring at least one transit time difference ($\Delta t_1$, $\Delta t_2$, $\Delta t_3$) between the time of the pulsed heating and the time at which a temperature ($T_{30}$, $T_{32}$) measured by a thermometer (30, 32) is at its maximum, and
(b) determining the parameter (B) from the at least one transit time difference ($\Delta t_1$, $\Delta t_2$, $\Delta t_3$).

3. The gas meter (10) according to one of the preceding claims, **characterised in that**

(a) it is a natural gas meter,
(b) the parameter (B) is a classification that encodes the production site of the natural gas and
(c) the detection of the parameter (B) using the at least one measured temperature ($T_{30}$, $T_{32}$, $T_{33}$) is a reading and/or interpolation of a characteristic diagram that assigns the parameter (B) to the at least one transit time difference ($\Delta t_1$, $\Delta t_2$, $\Delta t_3$).

4. The gas meter gas composition sensor (10) according to one of the preceding claims, **characterised in that** the detection of the parameter (B) using the at least one measured ($T_{30}$, $T_{32}$, $T_{33}$) is a reading and/or interpolation of a characteristic diagram that assigns the parameter (B) to the at least one transit time difference ($\Delta t_1$, $\Delta t_2$, $\Delta t_3$).

5. A natural gas network with

(i) a natural gas source (40),
(ii) a natural gas consumer (42) and
(iii) a natural gas pipeline (44) to transport natural gas (12) from the natural gas source (40) to the natural gas consumer (42),

**characterised by**
(iv) a natural gas meter (10) according to one of the preceding claims, which constitutes part of the natural gas pipeline (44).

6. The use of a gas meter according to one of the preceding claims 1 to 4 for determining a calorific value-weighted through-flow ($D_{BW}$) of natural gas.

## Revendications

1. Compteur de gaz, comportant

(a) un conduit de passage (14) et
(b) un capteur de composition de gaz (16) pour déterminer un paramètre (B) caractérisant la composition d'un fluide gazeux (12), comportant

(i) une source de chaleur (29) pour chauffer par impulsions le fluide (12) à mesurer,
(ii) au moins un thermomètre (30, 32, 33) pour mesurer en fonction du temps une température du fluide (12), et
(iii) une commande (22) réalisée pour automatiquement

- chauffer par impulsions le fluide (12) à mesurer au moyen de la source de chaleur (28),
- mesurer en fonction du temps la température ($T_{30}$, $T_{32}$, $T_{33}$) du fluide (12) au moyen dudit au moins thermomètre (30, 32, 33), et
- déterminer le paramètre (B) à l'aide de ladite au moins une température mesurée ($T_{30}$, T32, T33),

(iv) une distance ($x_{30}$) entre l'un au moins des thermomètres (30, 32) et la source de chaleur (29) étant au maximum de 100 $\mu$m, et
(v) la commande du capteur de composition de gaz (16) étant conçue pour automatiquement

- déterminer un débit du fluide gazeux (12),
- déterminer le paramètre (B) d'un fluide (12), caractérisant la composition du fluide gazeux (12), sous la forme d'un paramètre de contenu énergétique (B), et
- calculer le produit du débit (D) et du paramètre de contenu énergétique (B),

(c) une ouverture d'entrée (15), et
(d) une ouverture de sortie (17),

**caractérisé par**

(e) une zone à vitesse d'écoulement réduite (35) qui est

- est en communication fluidique avec l'ouverture d'entrée (15) et avec l'ouverture de sortie (17), et
- est réalisée de telle sorte qu'une vitesse d'écoulement moyenne ($v_{35}$) dans la zone à vitesse d'écoulement réduite (35) est au maximum un dixième d'une vitesse d'écoulement moyenne à travers l'ouverture d'entrée ($v_{15}$),

(f) le capteur de composition de gaz (16) étant disposé dans la zone à vitesse d'écoulement réduite (35).

2. Compteur de gaz selon la revendication précédente, **caractérisé en ce que** la commande (22) est conçue pour mettre en œuvre un procédé dans lequel la détermination du paramètre comprend les étapes suivantes consistant à :

(a) mesurer au moins une différence de temps de parcours ($\Delta t_1$, $\Delta t_2$, $\Delta t_3$) entre l'instant du chauffage par impulsions et l'instant où une température ($T_{30}$, $T_{32}$) mesurée par le thermomètre (30, 32) est à son maximum, et
(b) déterminer le paramètre (B) à partir de ladite au moins une différence de temps de parcours ($\Delta t_1$, $\Delta t_2$, $\Delta t_3$).

3. Compteur de gaz selon l'une des revendications précédentes, **caractérisé en ce que**

(a) il est un compteur de gaz naturel,
(b) le paramètre (B) est une classification codant le lieu d'extraction du gaz naturel, et

(c) la détermination du paramètre (B) à l'aide de ladite au moins une température mesurée ($T_{30}$, $T_{32}$, $T_{33}$) est une lecture et/ou une interpolation d'un champ caractéristique qui associe le paramètre (B) à ladite au moins une différence de temps de parcours ($\Delta t_1$, $\Delta t_2$, $\Delta t_3$).

4. Compteur de gaz selon l'une des revendications précédentes, **caractérisé en ce que** la détermination du paramètre (B) à l'aide de ladite au moins une température mesurée ($T_{30}$, $T_{32}$, $T_{33}$) est une lecture et/ou une interpolation d'un champ caractéristique qui associe le paramètre (B) à ladite au moins une différence de temps de parcours ($\Delta t_1$, $\Delta t_2$, $\Delta t_3$).

5. Réseau de gaz naturel, comprenant

   (i) une source de gaz naturel (40),
   (ii) un consommateur de gaz naturel (42) et
   (iii) un conduit de gaz naturel (44) pour acheminer le gaz naturel (12) de la source de gaz naturel (40) au consommateur de gaz naturel (42),

   **caractérisé par**
   (iv) un compteur de gaz naturel (10) selon l'une des revendications précédentes, qui fait partie du conduit de gaz naturel (44).

6. Utilisation d'un compteur de gaz selon l'une des revendications 1 à 4 pour déterminer un débit pondéré par la valeur calorifuge ($D_{BW}$) de gaz naturel.

Fig. 1b

Fig. 1a

Fig. 1

ballistischer Bereich

$10l = 0,68\ \mu m$

$500l = 34\mu m$

- D -

28

30

Fig. 2

Fig. 3b

Fig. 3a

14

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014008284 A1
- DE 69817875 T2
- US 20090049907 A1
- WO 2004065914 A1
- WO 200118500 A1
- DE 102012019657
- DE 102014010939
- DE 102011121213